# EUROPEAN PATENT APPLICATION

(11) **EP 2 554 188 A1**
(43) Date of publication of application: **06.02.2013**
(21) Application number: 10849028.5
(22) Date of filing: 09.12.2010
(51) Int. Cl.: A61L 31/00

(54) **ADHESION-PREVENTING MATERIAL**

(30) Priority: 31.03.2010 JP 2010082855
(71) Applicant: Hogy Medical CO., LTD., Tokyo 107-8615 (JP)
(72) Inventor: SOE Gilbu, Tokyo 107-8615 (JP); AOSHIMA Motonori, Tokyo 107-8615 (JP); INOUE Toshiki, Tokyo 107-8615 (JP); HASEGAWA Kiyotaka, Tokyo 107-8615 (JP)
(74) Representative: Cohausz & Florack
(86) International application number: PCT/JP2010/072141
(87) International publication number: WO 2011/121858

(57) **Abstract**

Provided are a novel carboxymethyl cellulose (CMC) structure and a method for producing the same. The CMC structure of the present invention is useful as a medical material and particularly as an adhesion-preventing material, and has good biocompatibility and bioabsorbability and controllable periods of functioning and dissolving, or has both effects for preventing adhesions and wound healing.

The CMC structure of the present invention is substantially composed of carboxymethyl celluloses, comprising an acid carboxymethyl cellulose and an alkaline metal carboxymethyl cellulose in a mixed state. The method of production of the present invention comprises subjecting the alkaline metal (or acid) carboxymethyl cellulose structure to an acid (or alkali) treatment, wherein the acid (or alkali) treatment is terminated before the alkaline metal (or acid) carboxymethyl cellulose is fully converted to the acid (or alkaline metal) carboxymethyl cellulose.

## Description

### TECHNICAL FIELD

The present invention relates to an adhesion-preventing material for preventing postoperative organ adhesions.
The present invention also relates to a carboxymethyl cellulose (CMC) structure and a method for producing the same. The CMC structure of the present invention can be used, for example, as a medical material, and more particularly for articles required to have properties to retain a shape for a certain period in the body and then to be absorbed/excreted such as adhesion-preventing material, base sheet for fibrin sealant, base for DDS, resorbable suture-reinforcing material, artificial dura, osteosynthesis material, and resorbable suture.

### BACKGROUND ART

In the clinical fields of cardiac surgery, orthopedic surgery, neurosurgery, abdominal surgery, obstetrics and gynecology, and the like, adhesions between an affected part and a surrounding living tissue caused by various surgeries or traumas are serious issues. The adhesion leads pain and/or dysfunction, and in some more serious cases, may cause intestinal obstruction or the like. Such a serious adhesion should require an additional surgery for detaching the adhesion. The adhesion also brings the problem of difficulty in reoperation for the primary disease. To prevent adhesions of living tissue, adhesion-preventing materials have been conventionally developed, that cover tissue liable to be adhered, to provide protection from adhesion. In fact, some adhesion-preventing materials such as oxidized regenerated cellulose fabric and sodium hyaluronate-carboxymethylcellulose membrane have already been put to practical use.

Specifically, to function in prevention of adhesion, an adhesion-preventing material should be present for a necessary period between an applied part (affected part) liable to be adhered to a surrounding tissue to act as a barrier for the applied part against the tissue and be finally degraded to be absorbed in the body. In other words, the adhesion-preventing material is required to be excellent in biocompatibility, bioabsorbability, timing control, and the like. However it is difficult to control the length of time that conventional adhesion-preventing materials exist in the body, for example, and they may stay in the body even after a concern about adhesions of a living tissue diminishes. Such a remaining barrier may be a burden for a patient.

To obviate these disadvantages, Patent Literature 1 discloses an adhesion-preventing material comprising a poorly water-soluble carboxymethyl cellulose having a dissolution half-life of 5 to 30 hours. The poorly water-soluble carboxymethyl cellulose can be produced, for example, as having a sponge texture by dissolving a water-soluble sodium carboxymethyl cellulose (sodium CMC) in distilled water to give a 1% by mass solution, adjusting the pH of the solution to 1.5 with 1N nitric acid, allowing the acidified solution to stand for 3 days at -20°C, and thawing at 25°C (Example 1). The poorly water-soluble carboxymethyl cellulose described in Patent Literature 1 is a product from a carboxymethyl cellulose through full conversion to acid CMC by an acid treatment, freezing and thawing. This adhesion-preventing material is characterized by having a controllable dissolution half-life. What is controllable is only a half-life. There is still a possibility that the adhesion-preventing material is not fully dissolved but remains in the body after fulfilled its role, and a patient may not be completely free from the burden.

Further, postoperative organ adhesions cause complications such as pain, infertility, and intestinal obstruction (ileus) to adversely affect postoperative QOL (quality of life) of a patient. Particularly in the field of obstetrics and gynecology, adhesions are serious issues that cause infertility. It is said that adhesions occur in about 90% of major surgeries in obstetrics and gynecology.

Methods of covering a wound site after surgery to prevent adhesions to a neighbor organ are often employed to prevent postoperative adhesions. For example, Patent Literature 2 discloses use of a water-soluble sodium carboxymethyl cellulose as an adhesion-preventing material for intraperitoneal injection in the form of 1% aqueous solution. Patent Literature 3 discloses use of a poorly water-soluble carboxymethyl cellulose (acid carboxymethyl cellulose) as an adhesion-preventing material in the form of film. Commercial adhesion-preventing materials are also known, including Seprafilm (Genzyme Japan K.K.; an adhesion-preventing material in the form of translucent film containing sodium hyaluronate and carboxymethyl cellulose in a ratio of 2:1 by weight) and Interceed (Johnson & Johnson K.K.; knitted fabric of regenerated oxidized cellulose). There is still a demand for an adhesion-preventing material having enhanced adhesion-preventing effects. For example, Patent Literature 3 describes that an aqueous solution of sodium carboxymethyl cellulose is confirmed to act as an adhesion-preventing material, but its effect is insufficient. Seprafilm requires cumbersome careful handling in application, because it will adhere to itself when wet or to a wet hand and is difficult to move from the hand to a predetermined site, and it may break due to excess drying under some storage conditions. Because of these shortcomings, Seprafilm cannot be expected to be sufficient to prevent adhesions.

### CITATION LIST

### PATENT LITERATURE

[Patent literature 1] Japanese Unexamined Patent Publication (Kokai) No. 2004-51531
[Patent literature 2] Japanese Unexamined Patent Publication (Kokai) No. 1-301624
[Patent literature 3] WO01/034214

### SUMMARY OF INVENTION

### TECHNICAL PROBLEM

To completely minimize a burden on a patient, it is desirable that a medical material (e.g., adhesion-preventing material) that has to be embedded for a given period in the body is quickly dissolved and absorbed in the body after the material no longer needs to function (e.g., for an adhesion-preventing material, when there is no possibility of adhesions of living tissues). Thus, control of a dissolution half-life only is insufficient for relieving the patient from the burden. It is desiralbe that a medical material has controllable periods of functioning and dissolving, as well as a dissolution half-life.
In addition, if an adhesion-preventing material can gradually release an alkaline metal CMC and particularly sodium CMC, which are known to have effects for wound healing, at an operated site, the adhesion-preventing material desirably has the effects of continuously promoting smooth wound healing together with adhesion-preventing effects.

The present inventor has extensively investigated to develop such a CMC structure, and found that a new CMC structure comprising a highly water-soluble alkaline metal CMC and a poorly water-soluble acid CMC can be produced by a partial acid treatment of a highly water-soluble alkaline metal CMC structure under conditions that keep its texture or a partial alkali treatment of an acid CMC structure under conditions that keep its texture (e.g., by the acid treatment of the alkaline metal CMC structure to the full extent and then the alkali treatment), and has the desired effects described above.
That is, the first object of the present invention is to provide a new CMC structure and a method for producing the same, which is useful as a medical material and particularly as an adhesion-preventing material, and has good biocompatibility and bioabsorbability and controllable periods of functioning and dissolving, and/or has effects both preventing adhesions and promoting wound healing.

The second object of the present invention is to provide an adhesion-preventing material having improved adhesion-preventing effects, compared with conventional barriers.
The present inventor has extensively investigated to solve the problem, and found that sodium carboxymethyl cellulose, which is known to have insufficient effects as an adhesion-preventing material, can in a fibrous form produce adhesion-preventing effects comparable to or better than that of Seprafilm, which is known to have the best effect among currently available adhesion-preventing materials on the market, thereby accomplished the present invention.
With respect to use of sodium carboxymethyl cellulose as an adhesion-preventing material, a common knowledge in the art at the time of the filing of the present application should be noted. The reason for Seprafilm comprising sodium hyaluronate and carboxymethyl cellulose at a ratio of 2:1 by weight is speculated to be that sodium carboxymethyl cellulose alone cannot produce sufficient adhesion-preventing effects and so is used together with sodium hyaluronate. In addition, so far as the present inventor knows, there is no commercially available adhesion-preventing material substantially composed of sodium carboxymethyl cellulose alone. This also clearly reflects the common knowledge in the art at the time of the filing of the present application, that sodium carboxymethyl cellulose cannot adequately prevent adhesions.
Therefore, according to the common knowledge, it was surprisingly and unexceptionally found that sodium carboxymethyl cellulose in a fibrous form could produce the adhesion-preventing effects as well or better than those of the commercial product Seprafilm.

### SOLUTION TO PROBLEM

The present invention relates to:
[1] a carboxymethyl cellulose structure substantially composed of carboxymethyl celluloses, comprising an acid carboxymethyl cellulose and an alkaline metal carboxymethyl cellulose in a mixed state;
[2] the carboxymethyl cellulose structure of [1], which is in a form of fiber sheet, film, or sponge;
[3] the carboxymethyl cellulose structure of [1] or [2], wherein the period of functioning in the body is from 5 hours to 6 months;
[4] an adhesion-preventing material comprising the carboxymethyl cellulose structure of any one of [1] to [3];
[5] a method for producing the carboxymethyl cellulose structure of any one of [1] to [3] or the adhesion-preventing material of [4], comprising subjecting an alkaline metal carboxymethyl cellulose structure to an acid treatment from the outside (particularly by immersing the structure in an acid solution), wherein the acid treatment (particularly immersion in the acid solution) is terminated before the alkaline metal carboxymethyl cellulose is fully converted to an acid carboxymethyl cellulose;
[6] a method for producing the carboxymethyl cellulose structure of any one of [1] to [3] or the adhesion-preventing material of [4], comprising subjecting an acid carboxymethyl cellulose structure to an alkali treatment from the outside (particularly by immersing the structure in an alkali solution), wherein the alkali treatment (particularly immersion in the acid solution) is terminated before the acid carboxymethyl cellulose is fully converted to an alkaline metal carboxymethyl cellulose;
[7] a method for controlling the period of functioning in the body of a carboxymethyl cellulose structure by controlling the period that an alkaline metal carboxymethyl cellulose structure is subjected to an acid treatment from the outside (particularly the period of immersing the structure in an acid solution); and
[8] a method for controlling the period of functioning in the body of a carboxymethyl cellulose structure by controlling the period that an acid carboxymethyl cellulose structure is subjected to an alkali treatment from the outside (particularly the period of immersing the structure in an alkali solution).
Hereinafter, these subjects of the present invention may also be collectively referred to as a first aspect of the present invention.

The present invention also relates to:
[1] an adhesion-preventing material substantially composed of alkaline metal carboxymethyl cellulose fibers;
[2] the adhesion-preventing material of [1] having a fabric weight of 10 to 300 g/m²; and
[3] the adhesion-preventing material of [1] or [2] having a molecular weight of 20000 to 2000000 Daltons.
Hereinafter, these subjects of the present invention may also be collectively referred to as a second aspect of the present invention.

### ADVANTAGEOUS EFFECTS OF INVENTION

According to the method of the present invention, a new CMC structure useful as a medical material and particularly as an adhesion-preventing material can be produced. The CMC structure produced by the method of the present invention has controlled periods of functioning and dissolving in the body, and is an excellent material for an article required to retain a shape for a given period in the body and then to be absorbed/excreted. The CMC structure also produces effects for simultaneously preventing adhesions and promoting wound healing.

The adhesion-preventing material of the present invention has better results in preventing adhesion than those of known commercial adhesion-preventing materials.

### BRIEF DESCRIPTION OF THE DRAWINGS

FIG. 1 is a photograph, instead of a drawing, showing the shape of a nonwoven fabric test piece before the treatment with hydrochloric acid in Example 1(1) (untreated).
FIG. 2 is a photograph, instead of a drawing, showing the shape of a nonwoven fabric test piece (treatment period with hydrochloric acid: 2 hours) after incubation for 10 days in an MEM medium.
FIG. 3 is a photograph, instead of a drawing, showing the shape of a nonwoven fabric test piece (treatment period with hydrochloric acid: 4 hours) after incubation for 10 days in an MEM medium.
FIG. 4 is a photograph, instead of a drawing, showing the shape of a nonwoven fabric test piece (treatment period with hydrochloric acid: 6 hours) after incubation for 10 days in an MEM medium.
FIG. 5 is a graph showing evaluated effects of the adhesion-preventing material of the present invention and a comparative commercial product (Seprafilm) when preventing adhesions in pigs (Example 4).
FIG. 6 is a graph showing evaluated effects of the adhesion-preventing material of the present invention and comparative products for preventing adhesions in mice (Example 7).
FIG. 7 is a graph showing evaluated effects of the adhesion-preventing material of the present invention and a comparative commercial product (Seprafilm) for preventing adhesions in mice (Example 9).

### DESCRIPTION OF EMBODIMENTS

The carboxymethyl cellulose (CMC) structure of the present invention can be produced, for example, by the method of the present invention, and is substantially composed of carboxymethyl celluloses comprising an acid carboxymethyl cellulose and an alkaline metal carboxymethyl cellulose in a mixed state.

As used herein, unless otherwise noted, the "carboxymethyl cellulose (CMC)" has the meaning including an easily water-soluble alkaline metal carboxymethyl cellulose (hereinafter, referred to as alkaline metal CMC) and a poorly water-soluble acid carboxymethyl cellulose (narrow carboxymethyl cellulose; hereinafter, referred to as acid CMC).
Examples of the alkaline metal CMC include sodium carboxymethyl cellulose (Na CMC) and potassium carboxymethyl cellulose.

Below, the first aspect of the present invention will be described, followed by the second aspect of the present invention.

### «First aspect of the present invention»

In the method of production of the present invention, an alkaline metal CMC (preferably Na CMC) structure is subjected to an acid treatment under conditions such that its texture is maintained. The acid treatment is terminated before the alkaline metal CMC is fully converted to an acid CMC, for example before the conversion reaches the center of the structure or to the inner section (hereinafter, referred to as the first production process). Alternatively, an acid CMC structure is subjected to an alkali treatment under conditions such that its texture is maintained. The alkali treatment is terminated before the acid CMC is fully converted to an alkaline metal CMC, for example before the conversion reaches to the center of the structure or to the inner section (hereinafter, referred to as the second production process). Alkaline metal CMC is easily soluble in water, and easily loses its texture by forming an aqueous solution by addition of water. However, in the method of production of the present invention, the alkaline metal CMC can maintain its texture during the acid or alkali treatment through use of a solvent comprising a lower alcohol, for example.

In the first production process, the acid treatment can be conducted, for example, by immersion of the structure in an acid solution, or application or spraying of an acid solution on to a surface of the structure. For example, the acid treatment by immersion is performed by immersing the alkaline metal CMC structure in an acid solution and removing it from the acid solution before the acid solution reaches the center of the structure or the inner section.
In the second production process, the alkali treatment can be similarly conducted by immersion of the structure in an alkali solution, or application or spraying of an alkali solution on to a surface of the structure in the same manner as the acid treatment in the first production process, except that an alkali solution is used instead of the acid solution. For example, the alkali treatment by immersion is performed by immersing the acid CMC structure in an alkali solution and removing it from the alkali solution before the alkali solution reaches the center of the structure or the inner section.

The alkaline metal CMC structure used in the first production process can be prepared, for example, by treating any CMC structure with an alkali to convert all CMC molecules to alkaline metal CMC molecules or by directly molding an alkaline metal CMC material into the structure. The acid CMC structure used in the second production process can be prepared, for example, by treating any CMC structure (e.g., an alkaline metal CMC structure produced by treating any CMC structure with an alkali to convert all CMC molecules to alkaline metal CMC molecules) with an acid to convert all CMC molecules to acid CMC molecules or by directly molding an acid CMC material into the structure.
Any CMC that can be used as a medical material can be used in the present invention. For example, the CMC has a degree of etherification of 0.5 to 1.5, and preferably 0.5 to 1, and has a molecular weight of 20000 to 2000000 Da, and preferably 20000 to 1000000 Da, based on the pullulan standard.

The first production process of the present invention can use any acid solution in the acid treatment of the alkaline metal CMC structure (e.g., immersion in the acid solution) provided that the acid solution can gradually penetrate into the alkaline metal CMC structure and can convert the alkaline metal CMC constructing the structure into an acid CMC. Examples of the acid include hydrochloric, sulfuric, nitric, and acetic acids. The concentration of the acid is generally 0.01 to 4.8 N, preferably 0.1 to 3.6 N, and more preferably 0.5 to 2.4 N. The alkaline metal CMC structure is composed of an easily water-soluble alkaline metal CMC, and thus the solvent used is preferably an aqueous alcohol mainly composed of a lower alcohol (e.g., methanol, ethanol, isopropanol) having an alcohol concentration of generally not less than 60%, preferably not less than 70%, and more preferably not less than 80%.

The second production process of the present invention can use any alkali solution in the alkali treatment of an acid CMC structure (e.g., immersion in the alkali solution) provided that the alkali solution can gradually penetrate into the acid CMC structure and can convert the acid CMC constructing the structure into an alkaline metal CMC. Examples of the alkali solution include aqueous solutions of sodium hydroxide, lithium hydroxide, potassium hydroxide, calcium hydroxide, barium hydroxide, and guanidine. The concentration of the alkali is generally 0.01 to 5 N, preferably 0.1 to 4 N, and more preferably 0.3 to 3 N. An easily water-soluble alkaline metal CMC is also contained in the product of the alkali treatment in the second production process, and thus the solvent used is preferably an aqueous alcohol mainly composed of a lower alcohol (e.g., methanol, ethanol, isopropanol) having an alcohol concentration of generally not less than 60%, preferably not less than 70%, and more preferably not less than 80%.

A period of immersion in the acid or alkali solution can be appropriately determined according to a form of the starting alkaline metal or acid CMC structure, and the kind, concentration, pH and temperature of the acid or alkali solution, and functions (i.e., periods of functioning and dissolving) of the product in the body required, and the like. For example, the period of immersion can be determined according to a simple pilot trial as described in the Examples below. More specifically, various periods of immersion were employed to prepare different samples (see, Examples 1(1) and 2(1)). These samples were subjected to an experiment about dissolution rate using an appropriate dissolution test liquid to select a sample having a desired dissolution profile (see, Examples 1(2) and 2(2)). Thus, a desired period of immersion can be determined.

The dissolution test liquid may be a test liquid that can reproduce conditions in the body as precisely as possible, or a test liquid that gives priority to fast evaluation (e.g., an MEM medium described in Example 1(2)).

In the CMC structure produced by the first production process of the present invention, the state of mixing of the acid and the alkaline metal CMCs can be appropriately determined according to a form of the CMC structure and conditions of the acid treatment with consideration to the functions required of the product in the body, and is not specifically limited. As is evident from the process of production, the CMC structure comprises the acid and the alkaline metal CMCs in a mixed state across the structure from the surface to the center. The acid and the alkaline metal CMCs may be distributed in such a state that the dominant component at the surface is the acid CMC and the dominant component at the center is the alkaline metal CMC and the content ratio gradually or intermittently varies from the surface to the center, or such a state that the outer part is composed of the acid CMC and the inner part is the alkaline metal CMC.

The CMC structure produced by the second production process of the present invention also has a similar state of mixing of the acid and the alkaline metal CMCs as above, except that a constitution of these CMCs is reversed. More specifically, the CMC structure comprises the alkaline metal and the acid CMCs in a mixed state across the structure from the surface to the center. The alkaline metal and the acid CMCs may be distributed in such a state that the dominant component at the surface is the alkaline metal CMC and the dominant component at the center is the acid CMC and a content ratio gradually or intermittently changes from the surface to the center, or such a state that the outer part is composed of the alkaline metal CMC and the inner part is the acid CMC.

In cases where the outer part composed of the acid CMC and the inner part composed of the alkaline metal CMC or the reversed constitution, the region between the outer and the inner parts (middle part) is not specifically limited. For example, the region between the outer and the inner parts may be composed of either the acid CMC or the alkaline metal CMC, or may comprise the acid CMC and the alkaline metal CMC in a mixed state.

The CMC structure of the present invention can have any form, but preferably a form suitable for use as a medical material. Examples of the form of the CMC structure include fiber sheets (e.g., knitted, woven, and nonwoven fabrics), films, and sponges.

When a CMC structure is immersed in a liquid, the liquid gradually penetrates into the structure. As used herein, the "outer part," the "middle part," and the "inner part" of the structure refer to the regions of the structure which the liquid penetrates at a first stage (outer part) and at a late stage (inner part) and a region between the outer and the inner parts (middle part), based on division of the course of penetration from the start of immersion in the liquid to the completion of penetration over the whole structure into the first, the middle, and the late stages.
As used herein, the "surface" of the structure refers to the region at which the liquid contacts with the structure upon immersion of the structure in the liquid.

For example, when the CMC structure is a film, the surface means a film surface. When the CMC structure is a fiber sheet (e.g., a knitted, woven, or nonwoven fabric), the surface means not an apparent sheet surface, but the surface of each constituent fiber.
Similarly for the outer and the inner parts, when the structure is a film, these parts directly mean the outer and the inner parts of the film. When the structure is a fiber sheet, these parts mean not the outer and the inner parts of the sheet, but the outer part and inner part of each constituent fiber.

In cases where the CMC structure of the present invention comprises an acid CMC and an alkaline metal CMC over the structure from the surface to the center, the CMC structure gradually releases the alkaline metal CMC having effects for wound healing and thus can produce effects both preventing adhesions and promoting wound healing simultaneously.

In cases where the outer part of the CMC structure of the present invention is composed of a poorly water-soluble acid CMC and the inner part is composed of an easily water-soluble alkaline metal CMC, when the CMC structure is placed in the body, for example, as an adhesion-preventing material, the outer acid CMC part gradually dissolves. When the dissolution reaches the extent that the inner alkaline metal CMC part becomes exposed, the inner part begins to quickly dissolve. From this point, the CMC structure dramatically changes its form. More specifically, the CMC structure having such a constitution of the present invention significantly changes its apparent overall dissolution rate between the period before the inner alkaline metal CMC becomes exposed (period of functioning) and the period after the inner alkaline metal CMC becomes exposed (period of dissolving), and thus the CMC structure having fulfilled its function will be eliminated quickly from the body.

In cases where the outer part of the CMC structure of the present invention is composed of an easily water-soluble alkaline metal CMC and the inner part composed of a poorly water-soluble acid CMC, the CMC structure is suitable for producing effects for promoting wound healing at an early stage after the CMC structure is placed. In this case, the easily water-soluble alkaline metal CMC having wound-healing effects quickly acts on an organ or the like at an early stage, and then the remaining inner acid CMC part will function for the desired period that it remains present.

For applications for preventing postoperative adhesions such as for using as an adhesion-preventing material, the CMC structure of the present invention can have various periods of functioning in the body from 5 hours to 6 months, which may be shorter or longer than the common period of preferred functioning of 2 days to 14 days. According to the present invention, the period of functioning can be adjusted to a desired length (preferably 1 day to 3 months, and more preferably 1 day to 1 month) according to the operation and condition. The presence of the CMC structure is preferably visually confirmed. The CMC structure of the present invention can be used, for example, in peritoneal and pelvic cavities.
For applications as a base sheet for fibrin sealant, the CMC structure of the present invention can have various periods of functioning in the body from 1 day to 3 months, which may be shorter or longer than a common period of preferred functioning of 2 days to 1 month. According to the present invention, the period of functioning can be adjusted to a desired length (preferably 1 day to 1 month) according to the operation and condition. The CMC structure of the present invention preferably has a sufficient mechanical strength (e.g., 1 MPa or more) and can be used, for example, in peritoneal, pelvic, and thoracic cavities. A fibrin sealant sheet can be prepared by loading a fibrin sealant such as thrombin/fibrinogen on the CMC structure of the present invention.
For applications as a base for drugs such as DDS, the CMC structure of the present invention can have various periods of functioning in the body from 1 day to 6 months, which may be shorter or longer than a common period of preferred functioning of 1 week to 1 month. According to the present invention, the period of functioning can be adjusted to a desired length (preferably 4 days to 3 months, and more preferably 1 week to 1 month) according to the operation and condition. The presence of the CMC structure is preferably visually confirmed. The CMC structure of the present invention can be used, for example, in peritoneal, pelvic, and thoracic cavities and the cranium.
For applications as a reinforcing patch for tissue junctions such as for using as a resorbable suture-reinforcing material, the CMC structure of the present invention can have various periods of functioning in the body from 3 days to 6 months, which may be shorter or longer than a common period of preferred functioning of 1 week to 2 months. According to the present invention, the period of functioning can be adjusted to a desired length (preferably 3 days to 3 months, and more preferably 3 days to 2 months) according to the operation and condition. The CMC structure of the present invention preferably has a sufficient mechanical strength (e.g., 1 MPa or more) and can be used, for example, in peritoneal, pelvic, and thoracic cavities and the cranium.
For applications as a dural substitute, such as for using as an artificial dura, the CMC structure of the present invention can have various periods of functioning in the body from 1 month to 24 months, which may be shorter or longer than a common period of preferred functioning of 4 months to 8 months. According to the present invention, the period of functioning can be adjusted to a desired length (preferably 3 months to 12 months) according to the operation and condition. The CMC structure of the present invention preferably has a sufficient mechanical strength (e.g., 1 MPa or more) and can be used in the cranium.
For applications as an osteosynthesis material for fixing bones each other such as use as a bolt, a nut, a screw, or a plate, the CMC structure of the present invention can have various periods of functioning in the body from 1 month to 24 months, which may be shorter or longer than a common period of preferred functioning of 3 months to 6 months. According to the present invention, the period of functioning can be adjusted to a desired length (preferably 1 months to 12 months, and more preferably 2 months to 9 months) according to the operation and condition. The CMC structure of the present invention preferably has a sufficient mechanical strength (e.g., 1 MPa or more) and can be used in bones throughout the body.
For applications as a resorbable suture, the CMC structure of the present invention can have various periods of functioning in the body from 1 week to 24 months, which may be shorter or longer than a common period of preferred functioning of 1 week to 2 months. According to the present invention, the period of functioning can be adjusted to a desired length (preferably 1 week to 12 months, and more preferably 1 week to 6 months) according to the operation and condition. The CMC structure of the present invention preferably has a sufficient mechanical strength (e.g., 1 MPa or more) and can be used in organs, tissues, the cranium and skin throughout the body.

### «Second aspect of the present invention»

The adhesion-preventing material of the present invention is substantially composed of alkaline metal carboxymethyl cellulose fibers, and more preferably composed of alkaline metal carboxymethyl cellulose fibers alone.
As used herein, unless otherwise noted, the "carboxymethyl cellulose (CMC)" has a meaning including an easily water-soluble alkaline metal carboxymethyl cellulose (hereinafter, referred to as alkaline metal CMC) and a poorly water-soluble acid carboxymethyl cellulose (narrow carboxymethyl cellulose; hereinafter, referred to as acid CMC), as described in the first aspect of the present invention.
The carboxymethyl cellulose used in the present invention comprises an alkaline metal carboxymethyl cellulose. Examples of the alkaline metal carboxymethyl cellulose include sodium carboxymethyl cellulose (Na CMC) and potassium carboxymethyl cellulose.

The alkaline metal carboxymethyl cellulose used in the present invention is in an anionic state at pH7.4 due to dissociation of a carboxymethyl group. Accordingly, the alkaline metal carboxymethyl cellulose can ionically bond to a basic protein such as chemokine or midkine at pH7.4.

The present invention can use any alkaline metal carboxymethyl cellulose that can be used as a medical material. For example, the alkaline metal carboxymethyl cellulose that can be used has a degree of etherification of 0.5 to 1.5, preferably 0.5 to 1, and more preferably 0.6 to 0.9, or has a molecular weight of 20000 to 2000000 Da, preferably 20000 to 1000000 Da, and more preferably 20000 to 500000 Da, based on the pullulan standard.

The alkaline metal carboxymethyl cellulose used in the present invention is only required to be fibrous and can be in any form. For example, the structure in a form of fiber may be used as is, or processed into the form of fiber sheet such as a knitted, woven, or nonwoven fabrics.
In cases where the alkaline metal carboxymethyl cellulose is in the form of fiber sheet, the fabric weight thereof is preferably 10 to 300 g/m².
In the particular case of the alkaline metal carboxymethyl cellulose in the form of woven fabric, the fabric weight thereof is preferably 40 to 300 g/m², and more preferably 80 to 250 g/m². In the case of the form of nonwoven fabric, the fabric weight thereof is preferably 10 to 150 g/m², and more preferably 15 to 80 g/m².

The alkaline metal carboxymethyl cellulose in the form of fiber used in the present invention is known per se (e.g., see, JP Pat. No. 3057446), and can be produced according to any known method, for example, by treating a natural, purified, or regenerated cellulose with aqueous ethanol containing sodium hydroxide, and then with aqueous ethanol containing monochloroacetic acid to give a carboxymethylated cellulose.

The mechanism of the alkaline metal carboxymethyl cellulose preventing adhesions in the adhesion-preventing material of the present invention is not fully understand now, but assumed, by the present inventor, to be as follows. It should be understood that the present invention should not be limited in the following mechanism.
The carboxymethyl cellulose used in the present invention comprises an alkaline metal carboxymethyl cellulose which is in an anionic state at pH 7.4 due to dissociation of a carboxymethyl group. Accordingly, the cellulose can ionically bond to a cytokine such as chemokine and midkine, which are basic proteins known to promote adhesion, thereby inhibiting their activities promoting adhesion.
An alkaline metal carboxymethyl cellulose is also known to have effects of hemostasis and promotion of cell adhesion and be used as a wound healing and hemostatic agent (JP Pat. No. 3057446). Considering that a pool of blood due to bleeding may be one of the factors in adhesion, the hemostatic effect of the cellulose may further enhance the effects for preventing adhesions.

### EXAMPLES

The present invention now will be further illustrated by, but is by no means limited to, the following Examples.

### <<EXAMPLE 1>>

### (1) Production of adhesion-preventing material in a form of nonwoven fabric

In a reaction vessel, 1 L of aqueous ethanol containing sodium hydroxide (4.2 mol/L of sodium hydroxide, 9.3 mol/L of ethanol) was added to 0.17 g of rayon nonwoven fabric (size: 10 cm by 10 cm, fabric weight: 17 g/m², thickness: 0.08 mm), and incubated for 17 hours at room temperature. To this, 615 mL of aqueous ethanol containing monochloroacetic acid (4.9 mol/L of monochloroacetic acid, 10.3 mol/L of ethanol) was added and incubated for 4 hours at 50°C. The product was washed with 70% methanol in water, and then 80% methanol in water, and neutralized to pH 6.0 to 8.0 with aqueous methanol containing hydrochloric acid (1.2 mol/L of hydrochloric acid, 90% methanol).
The product was washed with 80% methanol in water, and then 100% methanol, and dried to give a sheet (1). A major part of CMC molecules composing the sheet (1) was Na CMC. In the sheet (1), the degree of etherification was 0.83, and the molecular weight was 160000 Da.

The sheet (1) was cut into pieces (2 cm by 1 cm). Ten pieces were placed into each of eight 50 mL plastic tubes. To each tube was added 30 mL of aqueous methanol containing hydrochloric acid (1.2 mol/L of hydrochloric acid, 90% methanol). Tubes were incubated for different periods at room temperature: 0 minutes (i.e., not treated), 10 minutes, 20 minutes, 30 minutes, 1 hour, 2 hours, 4 hours, and 6 hours.
Incubated pieces were washed with 80% methanol in water, and then 100% methanol, and dried to give adhesion-preventing materials according to the present invention (hereinafter, referred to as samples).

### (2) Evaluation based on dissolution rate

Each sample was placed in a 50 mL plastic tube. To this was added 5 mL of 100% ethanol. The tube was sealed and the liquid was distributed evenly across the inside of the tube. The sample was dried through vacuum aspiration and then subjected to the following evaluation test.

An MEM medium used in a test for dissolution rate was prepared by adding 1 mL of 200 mmol/L L-glutamine (GIBCO 25030-081), 10 mL of fetal bovine serum (TRACE BIOSCIENCE 15-010-0500V), 1 mL of 5000 u/mL penicillin-5000 µg/mL streptomycin liquid (GIBCO 15070-063) to 100 mL of medium (GIBCO 10370-021). The medium contained a pH indicator, phenol red, and thus could indicate approximate pH by its color:
acidic (yellow)<<pH about 6.8 (orange) to about 8.0 (red)<<alkali (purple-red)

Each of the samples subjected to the treatment with hydrochloric acid for different treatment periods in Example 1(1) and disinfected with ethanol in Example 1(2) was placed in a 15 mL sample tube. To each tube was added 5 mL of MEM medium. Each tube was covered loosely so that air ventilation could occur, and placed in an incubator (37°C, 5% CO₂). As a color sample of MEM medium, a tube not containing a sample was also incubated in the same way.

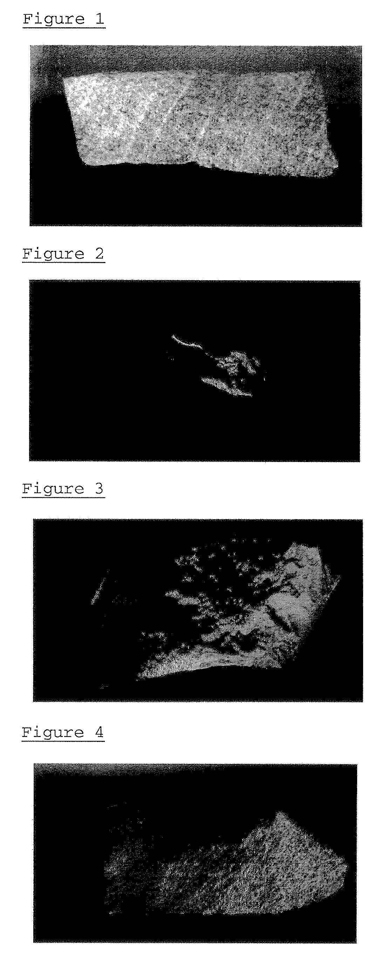

Just after of the addition of the MEM medium and on day 1, day 2, day 3, day 6, day 7, day 10, day 14, day 16, and day 17 after the addition of the MEM medium, each tube was taken from the incubator and examined for the appearance of the sample and color of the medium.
Degrees of dissolution of samples are shown in Table 1. A time shown in the sample column in Table 1 refers to the period of treatment with hydrochloric acid in Example 1(1).
Beside these chronological observations, samples were placed in a MEM medium for 10 days, taken from the MEM medium, and washed with 100% methanol. Shapes of samples thus treated are shown in FIGS. 2 (period of the treatment with hydrochloric acid: 2 hours), 3 (period of the treatment with hydrochloric acid: 4 hours), and 4 (period of the treatment with hydrochloric acid: 6 hours). As reference, FIG. 1 shows a shape of a piece of the sheet before subjected to the treatment with hydrochloric acid in Example 1(1).

**Table 1**

| [Sample]Day | | 0 | 1 | 2 | 3 | 6 | 7 | 10 | 14 | 16 | 17 |
|---|---|---|---|---|---|---|---|---|---|---|---|
| Untreated | | E | E | E | E | E | E | E | E | E | E |
| 10 | min | B | C | D | E | E | E | E | E | E | E |
| 20 | min | B | E | E | E | E | E | E | E | E | E |
| 30 | min | B | C | D | E | E | E | E | E | E | E |
| 1 | hr | A | A | B | C | E | E | E | E | E | E |
| 2 | hr | A | A | A | A | B | B | E | E | E | E |
| 4 | hr | A | A | A | A | A | A | D | D | E | E |
| 6 | hr | A | A | A | A | A | A | A | B | D | E |

| | | | | | | | | | | | |
|---|---|---|---|---|---|---|---|---|---|---|---|
| A: No swelling B: Swelling/clear C: Partially dissolving D: Almost dissolving E: Completely dissolving | | | | | | | | | | | |

As shown in Table 1, the more days that a sample was treated with hydrochloric acid (treatment of acidification), the larger the number of days required to dissolve it, with the exception that samples treated for 10 minutes took longer to dissolve than samples treated for 20 minutes.
In addition, as shown in FIGS. 1 to 4, the sample treated with hydrochloric acid for a short time became clear and almost dissolved (FIG. 2), while samples treated with hydrochloric acid for longer times (FIGS. 3 and 4) had the more similar appearance to the sample before treatment with hydrochloric acid (FIG. 1).

It is noted that there was a recognizable discrepancy between the result shown in FIG. 2 (period of the treatment with hydrochloric acid: 2 hours) where the sample almost dissolved but left residues and the result in Table 1 where the sample treated with hydrochloric acid for 2 hours was rated as E (completely dissolving) on day 10. The reason is assumed to be that the sample for chronological observation (sample in Table 1) was shaken each time conditions were observed, while photographic samples in FIGS. 2 to 4 were allowed to stay still for 10 days.

### «EXAMPLE 2»

### (1) Production of adhesion-preventing material in a form of film

The sheet (1) composed of Na CMC (degree of etherification: 0.83, molecular weight: 160000 Da), an intermediate case in Example 1(1), was dissolved in water to give an aqueous solution of Na CMC at a concentration of 50 mg/mL. 5 mL of the solution was applied to a slide glass (76 mm by 26 mm) and allowed to dry for two days and nights at room temperature. The resultant film was peeled from the slide glass and cut into pieces (2 cm by 1 cm). Ten pieces were placed in each of 50 mL plastic tubes.

To each tube was added 30 mL of aqueous methanol containing hydrochloric acid (1.2 mol/L of hydrochloric acid, 90% methanol). Tubes were incubated for different periods at room temperature: 0 hours (i.e., not treated) and 2 hours. Incubated pieces were washed with 80% methanol in water, and then 100% methanol, and dried to give adhesion-preventing material samples according to the present invention.

### (2) Evaluation based on dissolution rate

An evaluation based on the dissolution rate was conducted on film samples prepared in Example 2(1) together with nonwoven fabric samples prepared in Example 1(1) in the same way as described in Example 1(2).
Results are shown in Table 2.

**Table 2**

| **[Sample] Day** | **0** | 1 | **2** | **3** | **4** | **8** |
|---|---|---|---|---|---|---|
| **[Nonwoven fabric]** | | | | | | |
| **Untreated** | **E** | **E** | **E** | **E** | **E** | **E** |
| **2 hr** | **A** | **B** | **B** | **B** | **B** | **E** |

| **[Film]** | | | | | | |
|---|---|---|---|---|---|---|
| **Untreated** | **E** | **E** | **E** | **E** | **E** | **E** |
| **2 hr** | **A** | **B** | **C** | **C** | **D** | **E** |

| | | | | | | |
|---|---|---|---|---|---|---|
| A: No swelling B: Swelling/clear C: Partially dissolving D: Almost dissolving E: Completely dissolving | | | | | | |

### «EXAMPLE 3»

### (1) Production of adhesion-preventing material in a form of nonwoven fabric

The sheet (1) composed of Na CMC (degree of etherification: 0.83, molecular weight: 160000 Da), an intermediate case in Example 1(1), was cut into pieces (2 cm by 1 cm). Ten pieces were placed in each of eight 50 mL plastic tubes. To each tube was added 30 mL of aqueous methanol containing nitric acid (1.3 mol/L of nitric acid, 90% methanol). The tubes were shaken for 2 hours at room temperature to fully convert Na CMC to an acid CMC. The product was washed with 80% methanol in water, and then 100% methanol, and dried.
To each tube was added 30 mL of aqueous methanol containing sodium hydroxide (0.4 mol/L of sodium hydroxide, 77% methanol). The tubes were shaken for different periods at room temperature: 0 minutes (i.e., not treated), 10 minutes, 20 minutes, 30 minutes, 1 hour, 2 hours, 4 hours, and 6 hours. Products were washed with 80% methanol in water, and then 100% methanol, and dried to give adhesion-preventing materials according to the present invention.

### <<EXAMPLE 4>> Evaluation (1) of adhesion-preventing effects in pigs

### (1) Production of adhesion-preventing material

In a reaction vessel, 2.76 L of aqueous ethanol containing sodium hydroxide (2.9 mol/L of sodium hydroxide, 11 mol/L of ethanol) was added to 38 g of rayon nonwoven fabric (size: 20 cm by 100 cm, fabric weight: 19 g/m², thickness: 0.26 mm, ten sheets), and incubated for 17 hours at room temperature. To this was further added 1.65 L of aqueous ethanol containing monochloroacetic acid (3.4 mol/L of monochloroacetic acid, 13.4 mol/L of ethanol) and incubated for 6 hours at 50°C. The products were washed with 70% methanol in water, and then 80% methanol in water, and neutralized to pH 6.0 to 8.0 with aqueous methanol containing hydrochloric acid (1.2 mol/L of hydrochloric acid, 90% methanol).
The products were incubated for 2 hours in aqueous methanol containing hydrochloric acid (1.2 mol/L of hydrochloric acid, 90% methanol) at room temperature, washed with 80% methanol in water, and then 100% methanol, and dried to give adhesion-preventing materials according to the present invention.
The resultant sheets were cut into pieces having different dimensions (7.5 cm by 13 cm and 13 cm by 15 cm). Pieces were sterilized with electron beam irradiation and subjected to the following evaluation. These sheets had a degree of etherification of 0.88.

### (2) Evaluation for of adhesion-preventing effects

In this example, adhesion-preventing materials according to the present invention produced in Example 4 (1) (hereinafter, referred to as inventive sheet) were evaluated for adhesion-preventing effects using a pig. As a comparative sample, Seprafilm (Genzyme Japan K.K.; an adhesion-preventing material in a form of translucent film containing sodium hyaluronate and carboxymethyl cellulose at a ratio of 2:1 by weight) was used.
Each of six livestock piglets was incised along the median line under general anesthesia. One-third of the outer part of the left lobe of the liver was excised. An excised end was treated with an electrosurgical knife to stop bleeding, and covered with an inventive sheet (13 cm by 7.5 cm; 19 g/m²). The peritoneum was partially excised in dimensions of 8 cm by 8 cm at the left side of the median line, and covered with an inventive sheet (13 cm by 15 cm; 19 g/m²). The open abdomen was then closed.
For comparison, seven livestock piglets were operated on and received sheets of Seprafilm (12.7 cm by 7.35 cm and 12.7 cm by 14.7 cm) in the same way.

After two weeks of breeding, the piglets were operated on to create an incision at a position sufficiently far from the previous incision. In each piglet, all adhesions among organs were detached. Upon detachment of each adhesion, a degree of adhesion (by the number of grade) was rated according to the method of evaluation shown in Table 3 in Example 8 described below, and a contour of adhered sites was specified with a thread and captured in a photo together with a scale in order to calculate the area of the adhered site afterward. After the operation, areas of adhered sites were calculated using an area-calculating software. A score for each adhesion was calculated by multiplying the number of grade (1 to 4) with an adhered area (cm²). The total of scores of adhesions was considered as an adhesion score.

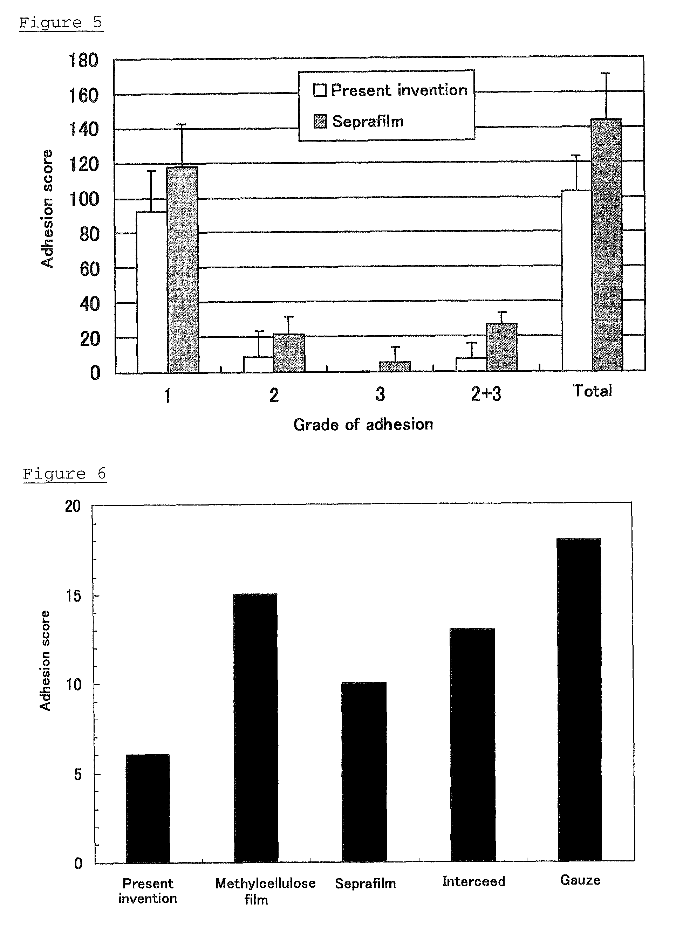

Results are shown in FIG. 5. In FIG. 5, p values (by the Mann-Whitney test) of grade 1, grade 2, grades 2 + 3, and the total were 0.1014 (not significant), 0.2240 (not significant), 0.0051(highly significant), 0.0082 (highly significant), respectively, but a p value of grade 3 could not be determined.
As can be seen from FIG. 5, the adhesion-preventing material of the present invention exhibited better adhesion-preventing effects than Seprafilm, particularly in middle to severe adhesions rated to grade 2 or 3 rather than in mild adhesions rated to grade 1. Therefore, the adhesion-preventing material of the present invention was thought to have better preventive effects against severe postoperative complications, such as intestinal obstruction, due to adhesions than Seprafilm.

### «EXAMPLE 5» Evaluation (2) of adhesion-preventing effects in pigs

This Example also used the inventive sheet produced in Example 4(1) and Seprafilm as a comparative sample.
Each of four livestock piglets was incised along the median line under general anesthesia. Knots were formed with 4-0 Prolene suture in the small intestine serosa at a position 50 cm down from the bottom of a retroperitoneal immobile section of the duodenum (suspensory muscle of duodenum) and a position a further 10 cm down from that position. A serosa between these knots was detached with a #80 sand cloth. The same operation was conducted at a section further 50 cm down therefrom. Two serosa-detached parts each having a length of 10 cm were thus formed. One serosa-detached part was covered with an inventive sheet (10 cm by 7.5 cm) and the other with a sheet of Seprafilm (10 cm by 7.35 cm). In a piglet, the serosa-detached part closer to the mouth was covered with an inventive sheet, and the other serosa-detached part closer to the anus was covered with a sheet of Seprafilm or vice versa.
After two weeks of breeding, piglets were operated on to create an incision at a position sufficiently far from the previous incision. In each piglet, all adhesions with serosa-detached parts in the small intestine were detached. An adhesion score was determined in the same way as in Example 4.

With respect to effects for preventing intestinal adhesions, the inventive sheet and Seprafilm had similar adhesion scores. There were no statistical difference between them according to the Mann-Whitney test result of p = 0.3836 (not significant). It is noted that all adhesions were rated as grade 1. The inventive sheet did however result in a smaller variation in effects for preventing adhesions, and was considered to be a better adhesion-preventing material that could stably produce adhesion-preventing effects.

### «EXAMPLE 6» Production of adhesion-preventing materials in forms of woven and nonwoven fabrics

Six sheets of Rayon woven fabric (size: 8.5 cm by 8.5 cm, fabric weight: 117 g/m²) weighing 5 g and forty sheets of Rayon nonwoven fabric (size: 8.5 cm by 8.5 cm, fabric weight: 17 g/m², thickness: 0.08 mm) weighing 5 g were treated in the following manner. In a reaction vessel, 1 L of aqueous ethanol containing sodium hydroxide (2.9 mol/L of sodium hydroxide, 11.0 mol/L of ethanol) was added to 5 g of either rayon fabric, and incubated overnight at room temperature. At an elevated temperature of 50°C, 0.6L of aqueous ethanol containing monochloroacetic acid (3.4 mol/L of monochloroacetic acid, 13.4 mol/L of ethanol) was addd and incubated with shaking for 4 hours at 50°C. The product was washed with 70% methanol in water twice, and then 80% methanol in water once, and neutralized to pH 6.0 to 8.0 with aqueous methanol containing hydrochloric acid (2.4 mol/L of hydrochloric acid, 80% methanol).
The product was washed with 80% methanol in water once, and then 100% methanol twice, and dried at 105°C to give an adhesion-preventing material according to the present invention (sheets of woven or nonwoven fabric). A major part of the CMC composing the sheet was Na CMC.
The resultant sheets were cut into pieces (5 mm by 1.2 cm). Pieces were sterilized with electron beam irradiation and subjected to the following evaluation. These sheets had a degree of etherification of 0.83 and a molecular weight of 160000 Da.

### «EXAMPLE 7» Evaluation for adhesion-preventing effects in mice

In this Example, the adhesion-preventing material according to the present invention (woven fabric sheet) produced in Example 6 was evaluated in mice. Gauze (cellulose), Seprafilm (Genzyme Japan K.K.; an adhesion-preventing material in a form of translucent film containing sodium hyaluronate and carboxymethyl cellulose at a ratio of 2:1 by weight), Interceed (Johnson & Johnson K.K.; oxidized regenerated cellulose fabric), and methylcellulose film were used for comparison.

A four-week-old mouse was anesthetized by intraperitoneal administration of 200 µL of an anesthetic, which was prepared by mixing 20 mL of Ketalar (Daiichi Sankyo Propharma Co., Ltd.) with a solution of 29.8 mg of xylazine in 3mL of phosphate-buffered saline (PBS). The mouse was confirmed to be in deep sleep under anesthesia and then shaved with an electric clippers at its belly.
The belly was disinfected with 70% ethanol and incised along the median line. The cecum was put out about 1 cm from the body with forceps. The cecum was grasped at about 5 mm from the top with a bipolar electric scalpel and nearly circumferentially processed for 1 second at 20 W.
The cecum was untreated (i.e., a control without a treatment for preventing adhesions on a processed part) or covered with one of the disinfected samples (5 mm by 1.2 cm) and returned into the body. The belly was closed with a silk thread.
After six days of breeding, the mice were euthanized by cervical dislocation. Each mouse was incised from a side of the belly with scissors with careful attention not to apply a pressure on the operated part, and the abdomen was opened widely.

After six days of burial in the body, only a gauze sample was confirmed to be remaining, and the present of the other samples could not be confirmed visually. Organs in the peritoneal cavity were grasped with forceps and evaluated for presence and extent of adhesions. A degree of adhesion was rated as follows:
0: there was no adhesion
1: there was an easy detachable adhesion
2: there was an adhesion requiring scissors to be detached.
When an adhesion had a length of 2cm or more, its score was doubled. The total of scores of adhesions to organs was used as an adhesion score.
Results are shown in FIG. 6.

The gauze sample caused adhesions in a wide area around the gauze sample and had the highest adhesion score. The samples of Seprafilm and Interceed, which are currently commercially available and in wide clinical use, had lower adhesion scores than that of the gauze sample.
The adhesion-preventing material sample according to the present invention had the lowest adhesion score. These results show that the adhesion-preventing material of the present invention has better adhesion-preventing effects than Seprafilm and Interceed.
The film sample composed of methylcellulose, which is a water-soluble polymer having a cellulose skeleton the same as the adhesion-preventing material of the present invention, had an equal adhesion score to Interceed.
Considering that the major difference between the adhesion-preventing material of the present invention and the methylcellulose film is the presence or absence of an anionic dissociating group, and that adhesions involve a basic protein having an isoelectric point such as chemokine and midkine, the adhesion-preventing material of the present invention was thought to prevent adhesions through ionic bonding with such a basic protein involved in adhesions to inhibit the function of the protein.

### «EXAMPLE 8» Evaluation of adhesion-preventing effects in pigs

In this Example, an adhesion-preventing material according to the present invention was produced in the same way as in Example 6, except that a sheet of rayon woven fabric having a size of about 13 cm by 15 cm (fabric weight: 200 g/m²) was used, and evaluated for preventing adhesions in pig. As a comparative sample, Seprafilm was used.

Each of three livestock piglets (line: LWD, female) was incised about 25 cm along the median line under general anesthesia. The median incision was opened with a retractor. One-third of the inner part of the left lobe of the liver was excised. The whole surface of an excised part was cauterized with an electrosurgical knife to stop bleeding (first operation). The peritoneum was partially excised in an area of dimensions of 8 cm by 8 cm at the left side of the median incision with an electrosurgical knife (second operation).
For a control piglet (without adhesion-preventing material), the peritoneum-fascia was sutured by a continuous suture, and the skin was sutured with a silk thread by an interrupted suture.
For a piglet with an adhesion-preventing material applied, the liver subjected to the first operation received a half-size sample (about 13 by 7.5 cm such that the excised end of the liver was covered. The excised area of the peritoneum subjected to the second operation was covered with a full-size sample (about 13 by 15 cm). The abdomen was closed in the same way as for the control piglet.

After two weeks of breeding, piglets were operated to create an incision at a position sufficiently far from the median incision under general anesthesia, and examined for the degree of adhesion. More specifically, each adhesion was detached with fingers or Metzenbaum scissors or the like, and evaluated according to the method of rating described in Table 3 to determine a grade of adhesion, and its size recoreded (area: cm²). The total of the products of grade and area of all adhesions was calculated and considered as an adhesion score.
A specific example of calculation of an adhesion score will be illustrated in Table 4 for the control piglet. In Table 4, respective sites shown in columns "adhered site 1" and "adhered site 2" on the same line refers to that these sites are adhered each other. Adhesion scores of piglets treated with different adhesion-preventing materials are shown in Tables 5 and 6.

**Table 3**

| Grade | Definition |
|---|---|
| 1 | Bluntly removable adhesion (removable with fingers). |
| 2 | Not bluntly removable adhesion (requiring Metzenbaum scissors or the like to remove). |
| 3 | Not bluntly removable adhesion (requiring Metzenbaum scissors or the like to remove and accompanying vascularization). |
| 4 | Adhesion inevitably causing actual damage to an organ through removal. |

**Table 4**

| | Adhered site | | | | |
|---|---|---|---|---|---|
| No. | 1 | 2 | Grade | Area | Score |
| 1 | Small intestine | Peritoneum | 1 | 9 | 9 |
| 2 | Greater omentum | Liver | 1 | 7 | 7 |
| 3 | Small intestine | Peritoneum | 2 | 19 | 38 |
| 4 | Liver | Median incision | 2 | 2 | 3 |
| 5 | Liver | Peritoneum, Diaphragm | 1 | 12 | 12 |
| 6 | Liver | Median incision, Peritoneum-excised part | 1 | 39 | 39 |
| 7 | Small intestine | Median incision, Peritoneum-excised part | 2 | 13 | 25 |
| 8 | Spleen | Liver | 3 | 3 | 10 |
| 9 | Excised end of the liver | Liver | 3 | 10 | 31 |
| 10 | Liver | Liver | 1 | 12 | 12 |

**Table 5**

| **Adhesion preventing-material** | **Adhesion score** | | **Adhesion area** | |
|---|---|---|---|---|
| | **Absolute value (Relative value)** | | **cm² (Relative value)** | |
| **Control (Not used)** | **187** | **(100)** | **126** | **(100)** |
| **Present invention** | **125** | **(67)** | **117** | **(93)** |
| **Seprafilm** | **107** | **(57)** | **89** | **(71)** |

**Table 6**

| **Adhesion preventing-material** | **Adhesion grade** | | | | |
|---|---|---|---|---|---|
| | **1** | **2** | **3** | **2+3 (Relative value)** | |
| **Control (Not used)** | **79** | **67** | **41** | **108** | **(100)** |
| **Present invention** | **109** | **16** | **0** | **16** | **(15)** |
| **Seprafilm** | **78** | **9** | **20** | **29** | **(27)** |

Compared with the control piglet, the adhesion-preventing material according to the present invention and Seprafilm for comparison both could reduce adhesions according to both indices of area and score of adhesion. The adhesion-preventing material according to the present invention and Seprafilm for comparison reduced the area of adhesion by 7% and 29%, respectively, and the score of adhesion by 33% and 43%, respectively (Table 3).
It is presumed that an adhesion of grade 1 will be gradually released over time. The onset of intestinal obstruction or the like due to postoperative adhesions varies from just after an operation to a few decades later. As such, an adhesion-preventing material will need to reduce adhesions of grade 2 or higher, while an adhesion of grade 1 that will be released over time is a matter of little significance for the adhesion-preventing material. If evaluated based on only adhesions of grade 2 or higher, the adhesion-preventing material according to the present invention reduced adhesions by 85% from the control, but Seprafilm for comparison reduced only by 73% (Table 4). The adhesion-preventing material according to the present invention was clinically confirmed to produce better effects than the known adhesion-preventing material.

### «EXAMPLE 9» Evaluation of adhesion-preventing effects in mice

In this Example, the adhesion-preventing materials according to the present invention (rayon woven fabric (100 g/m²) and rayon nonwoven fabric (19 g/m²)) produced according to Example 6 were evaluated for adhesion-preventing effects in mice. As comparative adhesion-preventing materials, Seprafilm and gauze were used.
A mouse was anesthetized (200 µL, intraperitoneal administration) and shaved. The belly of the mouse was incised about 1 cm. The cecum was put out about 1 cm from the body, grasped at about 5 mm from the top with bipolar forceps and nearly-circumferentially cauterized for 1 second at a coag mode (not to obstruct the cecum, left an uncauterized section of about 1 mm). The cecum was covered with one of the adhesion-preventing material samples (5 mm by 12 mm) and returned into the body. The incision was sutured with a silk thread.

After one week of breeding, the mice were euthanized. Each mouse was incised from the left lower part through the upper part to the right lower part of the abdomen. Adhesions of organs were detached and examined for the degree of adhesion. The degree of adhesion was scored as follows:
Easily removable adhesion: 1 point
Firm adhesion: 2 points
Firm adhesion having a length of 1 cm or more: 4 points
In cases of several adhesions formed in an organ, each adhesion was scored. The total of points in a mouse was considered the adhesion score of that mouse.

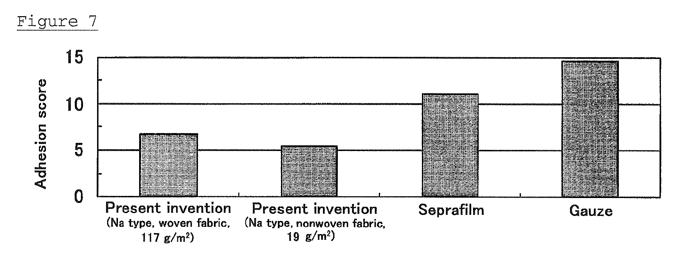

Results are shown in FIG. 7. Similar to results of Example 7, the gauze sample had the highest score of adhesion. Both woven and nonwoven adhesion-preventing materials according to the present invention had a lower score of adhesion than that of the Seprafilm sample. The adhesion-preventing material of the present invention was confirmed to have good adhesion-preventing effects whether it is in a form of woven or nonwoven fabric.

### INDUSTRIAL APPLICABILITY

The CMC structure of the present invention can be used, for example, as a medical material, and more particularly for articles required to have properties to retain a shape for a certain period in the body and then to be absorbed/excreted, such as adhesion-preventing material, base sheet for fibrin sealant, base for DDS, resorbable suture-reinforcing material, artificial dura, osteosynthesis material, and resorbable suture.
The adhesion-preventing material of the present invention can be used for preventing postoperative organ adhesions.
Although the present invention has been described with reference to specific embodiments, various changes and modifications obvious to those skilled in the art are possible without departing from the scope of the appended claims.

## Claims

1. An adhesion-preventing material substantially composed of alkaline metal carboxymethyl cellulose fibers.

2. The adhesion-preventing material according to claim 1, having a fabric weight of 10 to 300 g/m².

3. The adhesion-preventing material according to claim 1 or 2, having a molecular weight of 20000 to 2000000 Daltons.

4. A carboxymethyl cellulose structure substantially composed of carboxymethyl celluloses, comprising an acid carboxymethyl cellulose and an alkaline metal carboxymethyl cellulose in a mixed state.

5. The carboxymethyl cellulose structure according to claim 4, which is in a form of fiber sheet, film, or sponge.

6. The carboxymethyl cellulose structure according to claim 4 or 5, wherein the period of functioning in the body is from 5 hours to 6 months.

7. An adhesion-preventing material comprising the carboxymethyl cellulose structure according to any one of claims 4 to 6.

8. A method for producing the carboxymethyl cellulose structure according to any one of claims 4 to 6, comprising subjecting an alkaline metal carboxymethyl cellulose structure to an acid treatment from the outside, wherein the acid treatment is terminated before the alkaline metal carboxymethyl cellulose is fully converted to an acid carboxymethyl cellulose.

9. A method for producing the carboxymethyl cellulose structure according to any one of claims 4 to 6, comprising subjecting an acid carboxymethyl cellulose structure to an alkali treatment from the outside, wherein the alkali treatment is terminated before the acid carboxymethyl cellulose is fully converted to an alkaline metal carboxymethyl cellulose.

10. A method for controlling the period of functioning in the body of a carboxymethyl cellulose structure by controlling the period that an alkaline metal carboxymethyl cellulose structure is subjected to an acid treatment from the outside.

11. A method for controlling the period of functioning in the body of a carboxymethyl cellulose structure by controlling the period that an acid carboxymethyl cellulose structure is subjected to an alkali treatment from the outside.
